(19) Europäisches Patentamt
European Patent Office
Office européen des brevets

(11) Publication number: 0 319 906
A2

(12) EUROPEAN PATENT APPLICATION

(21) Application number: 88120345.9

(22) Date of filing: 06.12.88

(51) Int. Cl.4: C07D 215/56 , C07D 401/04 , A61K 31/47 , A61K 31/495

A request for correction on page 19, line 21 and page 37 the second line of table 6 has been filed pursuant to Rule 88 EPC. A decision on the request will be taken during the proceedings before the Examining Division (Guidelines for Examination in the EPO, A-V, 2.2).

(30) Priority: 11.12.87 JP 314790/87
07.11.88 JP 280610/88

(43) Date of publication of application:
14.06.89 Bulletin 89/24

(84) Designated Contracting States:
AT BE CH DE ES FR GB GR IT LI LU NL SE

(71) Applicant: Dainippon Pharmaceutical Co., Ltd.
25, Doshomachi 3-chome Higashi-ku
Osaka-shi, Osaka 541(JP)

(72) Inventor: Matsumoto, Jun-ichi
15-3, Shikanodai Higashi 2-chome
Ikoma-shi Nara-ken(JP)
Inventor: Minamida, Akira
1-8, Higashihagoromo 7-chome
Takaishi-shi Osaka-fu(JP)
Inventor: Hirose, Tohru
1152-13, Shimomatsu-cho
Kishiwada-shi Osaka-fu(JP)
Inventor: Nakano, Junji
6-6, Shikanodai Higashi 3-chome
Ikoma-shi Naka-ken(JP)
Inventor: Nakamura, Shinichi
12-20, Tsukawaki 1-chome
Takatsuki-shi Osaka-fu(JP)

(74) Representative: Kraus, Walter, Dr. et al
Patentanwälte Kraus, Weisert & Partner
Thomas-Wimmer-Ring 15
D-8000 München 22(DE)

(54) **Novel quinoline derivatives, processes for preparation thereof and antibacterial agent containing them.**

(57) 1-Substituted-6-fluoro-5-methyl-7-(piperazinyl or pyrrolidinyl)-1,4-dihydro-4-oxoquinoline-3-carboxylic acids and processes for preparation thereof. The acids are used for treatment of a bacterial infectious desease.

EP 0 319 906 A2

This invention relates to a novel quinoline derivative having very good antibacterial activity, processes for production thereof, and an antibacterial agent containing the quinoline derivative.

The compounds of this invention are quinoline derivatives represented by the following general formula (I)

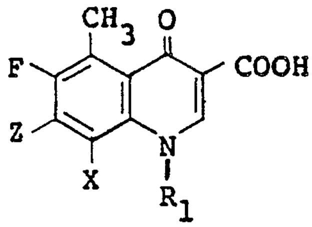

... (I)

wherein $R_1$ is a lower alkyl group having 1 to 5 carbon atoms, a cycloalkyl group having 3 to 6 carbon atoms, or an optionally substituted phenyl group, X is a hydrogen atom or a halogen atom, Z is

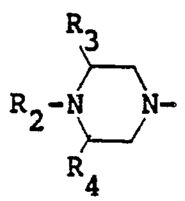

or

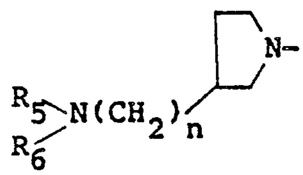

in which $R_2$, $R_3$, $R_4$, $R_5$ and $R_6$ are the same or different and each represents a hydrogen atom or a lower alkyl group having 1 to 5 carbon atoms, and n is 0 or 1,
or an ester thereof or a salt of the derivative or the ester.

In the above formula (I), examples of the halogen atom are fluorine and chlorine, and fluorine is especially preferred. The alkyl group may be linear or branched, and may be, for example, methyl, ethyl, propyl, isopropyl, butyl, sec-butyl, iso-butyl, t-butyl or pentyl.

The compound of formula (I), its ester, and salts of these are generically referred to as the compound of this invention.

The compounds of the invention may also exist as hydrates. Hence, these hydrates are also included within the compounds of this invention.

The compounds of the invention include those which have asymmetric carbon atoms [for example, when $R_3$ is an alkyl group having 1 to 5 carbon atoms in formula (I), the carbon atom to which the alkyl group is bonded], and therefore exist in optically active forms. Hence, they include D isomers, L isomers and mixtures thereof.

Some of the compounds of this invention have a plurality of asymmetric carbon atoms (for example, when both $R_3$ and $R_4$ are alkyl groups having 1 to 5 carbon atoms, the two carbon atoms to which $R_3$ and $R_4$ are bonded), and therefore can exist as stereoisomers having different configurations. These stereoisomers and their mixtures are also included within the compounds of this invention.

The esters of the compounds of formula (I) include, for example, aliphatic esters, particularly lower alkyl esters having 1 to 5 carbon atoms, such as methyl and ethyl esters; and esters whose alcohol moieties can be easily split off in vivo and converted to the compounds (I), for example acetoxymethyl esters, pivaloyloxymethyl esters, ethoxycarbonyloxyethyl esters, choline esters, aminoethyl esters (such as dimethylaminoethyl esters or piperidinoethyl esters), 5-indanyl esters, phthalidyl esters, and hydroxyalkyl esters (such as 2-hydroxyethyl esters and 2,3-dihydroxypropyl esters).

The salt of the compound of formula (I) or the salt of its ester should be understood as a salt formed between the compound of formula (I) or its ester with a pharmaceutically acceptable acid or base. Examples of the salts include salts of the compounds of formula (I) or their esters with inorganic acids such as hydrochloric acid and phosphoric acid; with organic acids such as acetic acid, lactic acid, oxalic acid, succinic acid, methanesulfonic acid, maleic acid, malonic acid and gluconic acid; with acidic amino acids such as aspartic acid and glutamic acid; with metals such as sodium, potassium, calcium, magnesium, zinc

and silver; with organic bases such as dimethylamine, triethylamine, dicyclohexylamine and benzylamine; and with basic amino acids such as lysine and arginine.

The prior art relating to pharmacologically effective compounds in this field will first be discussed briefly.

European Laid-Open Patent Publication No. 78,362 and its corresponding Japanese Laid-Open Patent Publication No. 74667/1983 disclose 1-cyclopropyl-6-fluoro-1,4-dihydro-4-oxo-7-piperazinyl-quinoline-3-carboxylic acids represented by the following formula (10)

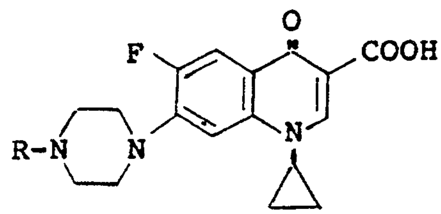

(10)

wherein R is H, $CH_3$, $C_2H_5$ or $HOCH_2CH_2$-,
and their pharmaceutically acceptable acid addition salts or hydrates.

European Laid-Open Patent Publication No. 113093 and its corresponding Japanese Laid-Open Patent Publication No. 130880/1984 disclose compounds represented by the following formula (11)

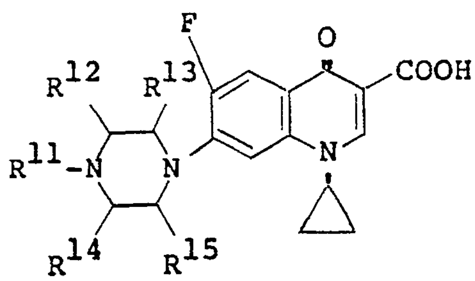

(11)

wherein $R^{11}$ is H or an optionally OH-substituted alkyl group having 1 to 12 carbon atoms, and $R^{12}$, $R^{13}$, $R^{14}$ and $R^{15}$ are the same or different and each represents an alkyl group having 1 to 4 carbon atoms, provided that at least one of $R^{12}$ to $R^{15}$ is the alkyl group,
their pharmaceutically accceptable acid addition salts, alkali metal or alkaline earth metal salts or hydrates.

U. S. Patent No. 4,556,658 and its corresponding Japanese Laid-Open Patent Publication No. 212474/1984 disclose compound represented by the following general formula (12)

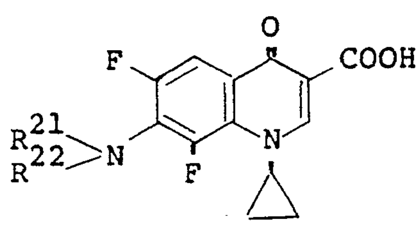

(12)

wherein

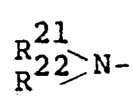

represents radicals including an optionaly substituted piperazinyl or pyrrolidinyl,
and its pharmaceutically useful salts.

U. S. Patent No. 4,665,079 and its corresponding Japanese Laid-Open Patent Publication No. 214773/1985 disclose compounds represented by the following general formula (13)

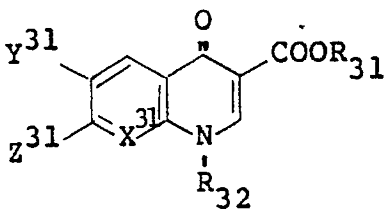

(13)

wherein $Z^{31}$ represents a specific pyrrolidinyl or a spiro-amino group, or other heterocyclic groups, $X^{31}$ is CH, CCl, CF N, etc., $Y^{31}$ is H, F, Cl, Br, $R_{31}$ is H, $C_{1-6}$ alkyl, or a cation, and $R_{32}$ is $C_{1-4}$ alkyl, vinyl, haloalkyl, $C_{2-4}$ hydroxyalkyl, or $C_{3-6}$ cycloalkyl,
or a pharmaceutically acceptable acid addition or base salt thereof.

The compounds of formulae (10), (11), (12) and (13) do not have a substituent at the 5-position of the quinoline ring as is seen from these formulae.

European Laid-Open Patent Publication No. 276700 and its corresponding Japanese Laid-Open Patent Publication No. 201170/1988 disclose compounds represented by the following general formula (14)

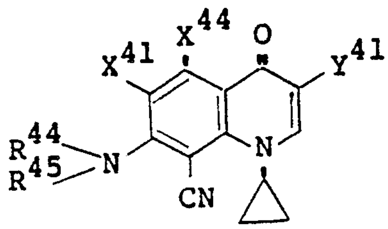

(14)

wherein $Y^{41}$ is COOH, CN, ester group or an amide group, $X^{41}$ is H, $NO_2$, alkyl or halogen, $X^{44}$ is H, halogen or alkyl, and

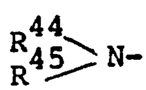

represents and optionally substituted heterocyclic group,
and pharmaceutically useful hydrates, salts, or esters thereof.

The compounds of this invention show excellent antibacterial activity and a broad antibacterial spectrum in in vitro tests. They are highly active against not only Gram-negative bacteria containing Pseudomonas aeruginosa and the genus Seratia, but also Gram-positive bacteria containing Streptococci and Methicillin-resistant Staphylococcus aureus, on which conventional quinolone-type antibacterial agents have relatively low activity. In addition, they are highly active against glucose-nonfermenters, anaerobes, and the genera Mycoplasma, Chlamydia and Mycobacterium, against which there are few effective drugs.

The compounds of this invention show an excellent protective effect in vivo on topical or systemic infections caused by various bacteria, and low toxicity in general toxicity tests on animals.

Therefore the compounds of this invention are useful as antibacterial agents administrable orally or by injection.

It is an object of this invention to provide novel quinoline derivatives of formula (I) and their pharmaceutically acceptable esters and salts.

Another object of this invention is to provide novel quinoline derivatives (I) and their pharmaceutially acceptable esters and salts which have excellent antibacterial activity in vitro and in vivo against both Gram-positvie and Gram-negative bacteria.

Still another object of this invention is to provide compounds which have a broad antibacterial spectrum and show excellent activity against the genera Mycoplasma and Chlamydia as well.

A further object of this invention is to provide a process for producing the novel compounds.

A still further object of this invention is to provide a pharmaceutical composition comprising an effective amount of a compound of formula (I) or its pharmaceutically acceptable ester or salt.

A yet further object of this invention is to provide a method of treating a bacterial infection of a warm-blooded animal, which comprises administering the compound or the pharmaceutical composition of the invention to the animal.

Additional objects of the invention along with its advantages will become apparent from the following description.

The processes for preparing the compounds of this invention will be described below.

A. Substitution reaction

The compounds of this invention can be produced by reacting a compound represented by the following general formula

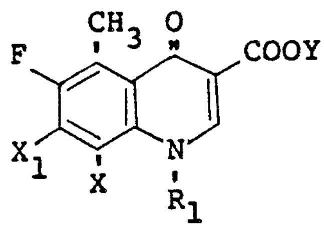

(II)

wherein $X_1$ is a halogen atom, Y is a hydrogen atom or a lower alkyl group having 1 to 5 carbon atoms, and $R_1$ and X are as defined with respect to formula (I),
with a compound represented by the following general formula

Z-H (III)

wherein Z is as defined above.

This reaction can be carried out by stirring the starting compounds (II) and (III) at 10 to 180 °C for 10 minutes to 24 hours in an inert solvent. Examples of the inert solvent include alcohols such as ethanol, ethers such as dioxane, tetrahydrofuran and 1,2-dimethoxyethane, aromatic hydrocarbons such as benzene, toluene and xylene, acetonitrile, dimethylformamide, dimethyl sulfoxide, pyridine and water.

Preferably, the above reaction is carried out in the presence of an acid acceptor using the starting compound of formula (III) in an equivalent or slightly excessive amount with regard to the starting compound (II). If desired, the starting compound (III) may be used in excess to make it serve concurrently as the acid acceptor. Examples of the acid acceptor are sodium hydrogen carbonate, sodium carbonate, potassium carbonate, triethylamine, diisopropylethylamine, 1,8-diazabicyclo[5.4.0]undecene-7 (DBU), and pyridine.

The starting compound (III) used in this reaction may, if possible, be used in a form protected by a protective group described in the reaction C hereinafter, and after the reaction, its protective group is removed in a customary manner.

The starting compound (II) can be prepared by the methods described in Referential Examples 1 to 3 or methods substantially in accordance with them.

B. Halogenation via diazonium salt

The compounds of this invention can be prepared by diazotizing a compound represented by the following general formula

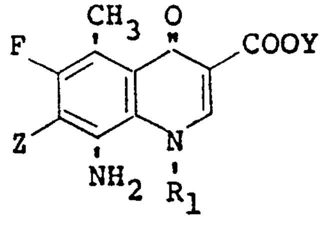

(IV)

wherein $R_1$, Y and Z are as defined above,
and halogenating the resulting diazonium salt.

The diazotizing reaction is carried out by stirring the compound (IV) and a diazotizing agent, usually under cooling, in water, an organic solvent such as ethanol, tetrahydrofuran, dioxane, acetonitrile or acetic acid, or a mixture, thereof with water in the presence or absence of an acid such as hydrochloric acid, sulfuric acid, phosphoric acid or nitrinc acid.

Examples of the diazotizing agent are nitrous acid, nitrites such as sodium nitrite, organic nitrous acid derivatives such as isoamnyl nitrite or t-butyl nitrite, and nitrosylsulfuric acid.

The halogenating reaction is carried out by stirring the resulting diazonium compound, with or without isolation, and a halogenating agent at 0-150 °C in such a solvent as described above and, in the case of fluorination, by decomposing the resulting diazonium salt.

Examples of the halogenating agent are cuprous chloride, copper-hydrochloric acid, tetrafluoroboric acid, hexafluorophosphoric acid and hexafluorosilicic acid.

The decomposition is carried out by heating the diazonium salt to 50-170 °C directly or in a diluent or organic solvent. Examples of the diluent are sand, barium sulfate and sodium fluoride. Examples of solvent are petroleum ether, cyclohexane, n-heptane, benzene, toluene, xylene, biphenyl, chloroform, tetrachloromethane, ethyl acetate, dioxane or quinoline.

The starting compound (IV) used in this reaction may, if possible, be used in a form protected by a protective group described in the reaction C hereinafter, and after the reaction, its protective group is removed in a customary manner.

The starting compound (IV) can be prepared by the method described in Referential Examples 4 to 5 or methods substantially in accordance with it.

C. Removal of amino protective group

The compound of this invention can be prepared by removing the protective group solvolyzing (also hydrolyzing) or reducing a compound represented by the following general formula

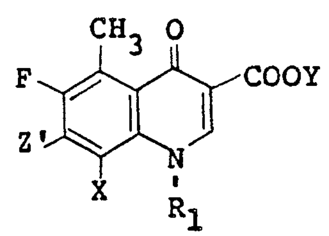

(V)

wherein $R_1$, X and Y are as defined above, and Z′ is

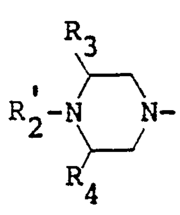

or

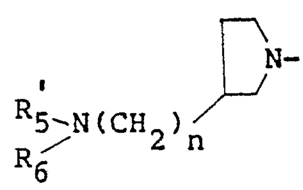

in which $R_2'$ and $R_5'$ represent a protective group, and $R_3$, $R_4$, $R_6$ and n are as defined above.

The protective group may be any protective group which can be removed without destroying the structure of the compounds of this invention formed by the reaction. Groups which are normally used as protective groups for the amino group in the field of chemistry of peptides, aminosugars, nucleic acids or beta-lactam compounds may be used in this invention.

The amino protective groups may be split off by solvolysis (including hydrolysis) or reduction depending upon the properties of the protective groups.

Specific examples of the protective groups capable of being eliminated by solvolysis include acyl

groups such as formyl, acetyl and trifluoroacetyl; substituted or unsubstituted alkoxycarbonyl groups such as ethoxycarbonyl, t-butoxycarbonyl, benzyloxycarbonyl, p-methoxybenzyloxycarbonyl and beta-(p-toluenesulfonyl) ethoxycarbonyl; a trityl group, a trimethylsilyl group, an o-nitrophenylsulfenyl group; a diphenylphosphinyl group; and a tetrahydropyranyl group.

This reaction is carried out in a solvent at 0 to 150 °C in the presence or absence of a catalyst such as an acid or base.

Examples of the acid are inorganic acids such as hydrochloric acid, hydrobromic acid, sulfuric acid and phosphoric acid; organic acids such as acetic acid, trifluoroacetic acid, formic acid, and toluenesulfonic acid; Lewis acids such as boron tribromide and aluminum chloride. Examples of the base are hydroxides such as sodium hydroxide and barium hydroxide, carbonates such as sodium carbonate and potassium carbonate; alkali metal alkoxides such as sodium methoxide and sodium ethoxide; and sodium acetate. Usually, water is used as the solvent. Depending upon the property of the compound, another solvent such as ethanol, dioxane, ethylene glycol dimethyl ether, benzene or acetic acid, or a mixed solvent of such a solvent with water may be used.

Examples of protective groups that may be eliminated by reduction include arylsulfonyl groups such as p-toluenesulfonyl; a methyl group substituted by phenyl or benzyloxy, such as benzyl, trityl or benzyloxymethyl; arylmethoxycarbonyl groups such as benzyloxycarbonyl and p-methoxybenzyloxycarbonyl; and halogenoethoxycarbonyl groups such as beta,beta,beta-trichloroethoxycarbonyl and beta-iodoethoxycarbonyl groups.

This reaction uses different reaction conditions depending upon the property of the protective group to be eliminated. For example, it is carried out by treating the compound with a hydrogen stream in an inert solvent at 10 to 60 °C in the presence of a catalyst such as platinum, palladium or Raney nickel (catalytic reduction); or treating it with metallic sodium in liquid ammonia usually at -50 to -20 °C; or by treating it with a metal such as zinc in acetic acid or in an alcohol such as methanol. Examples of the solvent in the catalytic reduction may include ethylene glycol dimethyl ether, dioxane, dimethylformamide, ethanol, ethyl acetate and acetic acid.

The starting compound (V) is a novel compound, and can be prepared by the reactions A and B above.

Where the compounds of this invention obtained by the above processes are esters, they can be converted to compounds of formula (I) by hydrolyzing the ester moiety in a customary manner. If required, the compounds of formula (I) may be esterified in a customary manner to form esters of the compounds of formula (I).

Pharmaceutically acceptable salts of the compounds of formula (I) or their esters may be produced by treating the compounds of formula (I) or esters thereof with acids, or by treating the compounds (I) with bases or metal salts. Acids suitable for salt formation include, for example, hydrochloric acid, phosphoric acid, acetic acid, lactic acid, oxalic acid, succinic acid, methanesulfonic acid, maleic acid, malonic acid, gluconic acid, aspartic acid and glutamic acid. Bases or metal salts suitable for salt formation include, for example, metal hydroxides such as sodium hydroxide and potassium hydroxide, metal carbonates such as sodium carbonate and potassium carbonate, zinc chloride, zinc sulfate, zinc nitrate and silver nitrate.

The compounds of this invention prepared as stated above are isolated and purified in a customary manner, and depending upon the isolating and purifying conditions, may be obtained in the form of a salt or a free acid. They may be converted into each other to produce the compounds of this invention in the desired forms.

The stereoisomers of the compounds of this invention can be isolated by a conventional method such as fractional crystallization or chromatography. It is possible to produce compounds of this invention having a specific configuration by the reactions described above using the starting compounds having a corresponding configuration.

The optically active isomers of the compounds of this invention can be separated by known methods.

The compounds (I) thus obtained, their esters, and salts of these are all new compounds and valuable as antibacterial agents since they have very high antibacterial activity. The compounds (I) and their salts can be used not only as medicines for man and animals, but as fish medicines, agricultural chemicals and food preservatives. The esters of the compounds (I) are of course valuable as starting materials for synthesizing the compounds (I). When the esters can be easily transformed into the compounds (I) *in vivo*, they can exhibit an equivalent effect and are also useful as antibacterial agents.

When the compounds of this invention are used as antibacterial agents for man, it is recommended that they be administered in a dose of 5 mg to 5 g per day once or several times daily, although the dose may be varied depending upon the age, body weight and symptom of a patient, the administration route, etc. The compounds may be administered orally or parenterally.

The compounds of this invention may be administered in their as-obtained powder form, but they are

usually administered in the form of a pharmaceutical preparation together with pharmaceutically acceptable adjuvants. Specific examples of the pharmaceutical preparations are tablets, solutions, capsules, granules, fine granules, pellets, powders, syrups, injections, and ointments. These pharmaceutical preprations are prepared by methods known per se. Adjuvants for oral administration are those which are commonly used in the field of formulating pharmaceutical preparations and do not react with the compounds of the invention, such as starch, mannitol, crystalline cellulose, CMC Na, water, ethanol, etc. Adjuvants for injections are those commonly used in the field of injection such as water, isotonic sodium chloride solution, glucose solution and transfusion solution.

The above liquid preparations and ointments can also be used for local treatments in oto-rhinolaryngology or ophthalmology.

The following examples illustrate the production of the compounds of this invention more specifically.

Referential Example 1

1-Cyclopropyl-6,7,8-trifluoro-5-methyl-1,4-dihydro-4-oxoquinoline-3-carboxylic acid:

(1) 32 g of 2,3,4,5-tetrafluoro-6-methylbenzoic acid, a known compound, was treated with thionyl chloride (27.5 g) and dimethylformamide (0.5 ml) to give 30.8 g of 2,3,4,5-tetrafluoro-6-methylbenzoyl chloride. b.p. 77-80 °C (19 mmHg).

(2) The above compound (30.8 g) was reacted with diethyl ethoxymagnesiummalonate to give diethyl 2,3,4,5-tetrafluoro-6-methylbenzoylmalonate (43.9 g). Water (400 ml) and p-toluenesulfonic acid monohydrate (0.40 g) were added to the above ester, and the mixture was refluxed for 2 hours. After cooling, the mixture was neutralized with saturated aqueous sodium bicarbonate solution and extracted with chloroform. The extract was dried and evaporated under reduced pressure, and the residue was purified by silica gel chromatography to give ethyl 2,3,4,5-tetrafluoro-6-methylbenzoylacetate (23.5 g). b.p. 122-128 °C (5 mmHg).

(3) A mixture of the above compound (6.0 g), acetic anhydride (5.5 g) and ethyl orthoformate (4.8 g) was refluxed for 3 hours. The mixture was evaporated to dryness under reduced pressure. The residue was dissolved in isopropyl ether (70 ml) and cyclopropylamine (1.23 g) was added at room temperature, and the mixture was stirred for 1 hour. n-Hexane was added to the mixture and the precipitated crystals were filtered off. Recrystallization from n-hexane gave ethyl 3-cyclopropylamino-2-(2,3,4,5-tetrafluoro-6-methylbenzoyl)acrylate (7.4 g). m.p. 76-77 °C.

(4) The above compound (7.40 g) was dissolved in dry tetrahydrofuran (70 ml) and potassium t-butoxide (2.4 g) was added with ice-cooling. After stirring for 1 hour, ice-water was added to the mixture and the precipitated crystals were collected. Recrystallization from ethyl acetate to give ethyl 1-cyclopropyl-6,7,8-trifluoro-5-methyl 1,4-dihydro-4-oxoquinoline-3-carboxylate (5.5 g). m.p. 176-177 °C.

(5) To the above compound (5.2 g) was added a mixed solution (50 ml) of concentrated sulfuric acid, glacial acetic acid and water (1:8:6) and the mixture was heated at 100 °C for 1 hour with stirring. After cooling, water was added and the precipitated crystals were collected. Recrystallization from acetonitrile gave 1-cyclopropyl-6,7,8-trifluoro-5-methyl-1,4-dihydro-4-oxoquinoline-3-carboxylic acid (4.2 g). m.p. 242-243 °C.

Referential Example 2

6,7,8-trifluoro-1-(2,4-difluorophenyl)-5-methyl-1,4-dihydro-4-oxoquinoline-3-carboxylic acid:

(1) In the same manner as described in Referential Example 1 (3), ethyl 2,3,4,5-tetrafluoro-6-methylbenzoylacetate (6.0 g), acetic anhydride (5.5 g), ethyl orthoformate (4.8 g) and 2,4-difluoroaniline (2.78 g) were allowed to react to give ethyl 3-(2,4-difluoroanilino)-2-(2,3,4,5-tetrafluoro-6-methylbenzoyl)-acrlate (7.9 g), which was recrystallized from n-hexane. m.p. 69-71 °C.

(2) The above compound (7.5 g) was treated with potassium t-butoxide in the same manner as described in Referential Example 1 (4) to give ethyl 6,7,8-trifluoro-1-(2,4-difluorophenyl)-5-methyl-1,4-dihydro-4-oxoquinoline-3-carboxylate (6.5 g), which was recrystallized from ethyl acetate. m.p. 152-154 °C.

(3) The above compound (6.0 g) was treated in the same manner as described in Referential Example 1 (5) to give 6,7,8-trifluoro-1-(2,4-difluorophenyl)-5-methyl-1,4-dihydro-4-oxoquinoline-3-carboxylic acid (5.1 g). m.p. 217-219 °C.

Referential Example 3

1-Cyclopropyl-6,7-difluoro-5-methyl-1,4-dihydro-4-oxoquinoline-3-carboxylic acid;

(1) 200 g of 2,4,5,6-tetrafluoroisophthalonitrile, a known compound, was reacted with diethyl malonate (160 g) in the presence of potassium fluoride to give 2,5,6-trifluoro-4-(diethoxycarbonylmethyl)-isophthalonitrile (332 g) as an oil.

(2) The above compound (181 g) was hydrolyzed with 60 % sulfuric acid to give 4-carboxymethyl-2,5,6-trifluoroisophthalic acid (125 g). m.p. 212-214 °C.

(3) The above compound (28 g) in dimethyl sulfoxide was heated at 140 °C in the presence of triethylamine to give 3,4,6-trifluoro-2-methylbenzoic acid (13 g). m.p. 114-115 °C.

(4) The above compound (24 g) was treated with thionyl chloride to give 3,4,6-trifluoro-2-methylbenzoyl chloride. b.p. 98-100 °C (40 mmHg).

This compound was reacted with diethyl sodium malonate in dry toluene to give diethyl (3,4,6-trifluoro-2-methylbenzoyl)-malonate.

To this compound were added water and a catalytic amount of p-toluenesulfonic acid, and the mixture was refluxed for 4 hours to give ethyl (3,4,6-trifluoro-2-methylbenzoyl)-acetate (15.2 g) as an oil. b.p. 112-116 °C (3 mmHg).

(5) The above compound (5.0 g) was treated with ethyl orthoformate and acetic anhydride to give ethyl 3-ethoxy-2-(3,4,6-trifluoro-2-methylbenzoyl)acrylate, which was then reacted with cyclopropylamine to give ethyl 3-cyclopropylamino-2-(3,4,6-trifluoro-2-methylbenzoyl)acrylate (4.5 g). m.p. 79-80 °C.

(6) The above compound (4.25 g) was dissolved in tetrahydrofuran (35 ml) and potassium t-butoxide (1.53 g) was added under ice-cooling. The mixture was stirred at room temperature for 15 minutes, ice water (100 ml) was added, and the precipitated crystals were collected. Chloroform and water were added to the crystals, and the chloroform layer was separated and dried over anhydrous sodium sulfate. Chloroform was evaporated under reduced pressure and the residue was recrystallized from acetonitrile to give ethyl 1-cyclopropyl-6,7-difluoro-5-methyl-1,4-dihydro-4-oxoquinoline-3-carboxylate (3.59 g). m.p. 198-200 °C.

(7) A mixture of the above compound (3.30 g), glacial acetic acid (8 ml), water (6 ml) and concentrated sulfuric acid (1 ml) was heated at 120 °C for 1 hour with stirring. After cooling, water (50 ml) was added to the mixture and the precipitated crystals were collected and washed with water. Recrystallization from acetonitrile gave 1-cyclopropyl-6,7-difluoro-5-methyl-1,4-dihydro-4-oxoquinoline-3-carboxylic acid (2.85 g). m.p. 229-231 °C.

Example 1

1-Cyclopropyl-6,8-difluoro-5-methyl-7-(cis-3,5-dimethyl-1-piperazinyl)-1,4-dihydro-4-oxoquinoline-3-carboxylic acid.

A mixture of 1-cyclopropyl-6,7,8-trifluoro-5-methyl-1,4-dihydro-4-oxoquinoline-3-carboxylic acid (0.50 g), cis-2,6-dimethylpiperazine (0.61 g) and pyridine (30 ml) was refluxed for 4 hours. The solvent was evaporated. Ethanol was added to the residue and the precipitated crystals were filtered off. The crystals were dissolved in 3 % aqueous acetic acid. The solution was treated with activated carbon, adjusted to pH ca 8.0 with 1N aqueous sodium hydroxide and cooled with ice. The precipitated crystals were collected, washed with water, and dried to give 1-cyclopropyl-6,8-difluoro-5-methyl-7-(cis-3,5-dimethyl-1-piperazinyl)-1,4-dihydro-4-oxoquinoline-3-carboxylic acid (0.42 g). m.p. 200-202 °C. [hydrochloride, m.p. 290-295 °C (decompn)]

Example 2

1-Cyclopropyl-6,8-difluoro-5-methyl-7-(3-methyl-1-piperazinyl)-1,4-dihydro-4-oxoquinoline-3-carboxylic acid:

1-Cyclopropyl-6,7,8-trifluoro-5-methyl-1,4-dihydro-4-oxoquinoline-3-carboxylic acid (0.50 g) and 2-methylpiperazine (0.54 g) were allowed to react in the same manner as described in Example 1 to give 1-cyclopropyl-6,8-difluoro-5-methyl-7-(3-methyl-1-piperazinyl)-1,4-dihydro-4-oxoquinoline-3-carboxylic acid (0.40 g). m.p. 188-191 °C.

Example 3

1-Cyclopropyl-6,8-difluoro-5-methyl-7-(1-piperazinyl)-1,4-dihydro-4-oxoquinoline-3-carboxylic acid:

1-Cyclopropyl-6,7,8-trifluoro-5-methyl-1,4-dihydro-4-oxoquinoline-3-carboxylic acid (0.50 g) and anhydrous piperazine (0.50 g) were allowed to react in the same manner as described in Example 1 to give 1-cyclopropyl-6,8-difluoro-5-methyl-7-(1-piperazinyl)-1,4-dihydro-4-oxoquinoline-3-carboxylic acid (0.42 g). m.p. 210-21 °C.

Example 4

7-(3-Amino-1-pyrrolidinyl)-1-cyclopropyl-6,8-difluoro-5-methyl-1,4-dihydro-4-oxoquinoline-3-carboxylic acid:

1-Cyclopropyl-6,7,8-trifluoro-5-methyl-1,4-dihydro-4-oxoquinoline-3-carboxylic acid (0.50 g) and 3-aminopyrrolidine (0.7 ml) were alowed to react in the same manner as described in Example 1 to give 7-(3-amino-1-pyrrolidinyl)-1-cyclopropyl-6,8-difluoro-5-methyl-1,4-dihydro-4-oxoquinoline-3-carboxylic acid (0.43 g). m.p. 232-234 °C.

Example 5

7-(3-Aminomethyl-1-pyrrolidinyl)-1-cyclopropyl-6,8-difluoro-5-methyl-1,4-dihydro-4-oxoquinoline-3-carboxylic acid:

1-Cyclopropyl-6,7,8-trifluoro-5-methyl-1,4-dihydro-4-oxoquinoline-3-carboxlic acid (0.50 g) and 3-aminomethyl-pyrrolidine (0.50 g) wre allowed to react in the same manner as described in Example 1 to give 7-(3-aminomethyl-1-pyrrolidinyl)-1-cyclopropyl-6,8-difluoro-5-methyl-1,4-dihydro-4-oxoquinoline-3-carboxylic acid (0.42 g). The compound melted at 109-112 °C and solidified and then melted at 178-179 °C.

Example 6

1-Cyclopropyl-6,8-difluoro-5-methyl-7-(4-methyl-1-piperazinyl)-1,4-dihydro-4-oxoquinoline-3-carboxylic acid:

1-Cyclopropyl-6,7,8-trifluoro-5-methyl-1,4-dihydro-4-oxoquinoline-3-carboxylic acid and 1-methylpiperazine were allowed to react in the same manner as described in Example 1 to give 1-cyclopropyl-6,8-difluoro-5-methyl-7-(4-methyl-1-piperazinyl)-1,4-dihydro-4-oxoquinoline-3-carboxylic acid. m.p. 228-229 °C.

Example 7

6,8-Difluoro-1-(2,4-difluorophenyl)-5-methyl-7-(1-piperazinyl)-1,4-dihydro-4-oxoquinoline-3-carboxylic acid:

6,7,8-Trifluoro-1-(2,4-difluorophenyl)-5-methyl-1,4-dihydro-4-oxoquinoline-3-carboxylic acid (0.50 g) and

anhydrous piperazine (0.50 g) were allowed to react in the same manner as described in Example 1 to give 6,8-difluoro-1-(2,4-difluorophenyl)-5-methyl-7-(1-piperazinyl)-1,4-dihydro-4-oxoquinoline-3-carboxylic acid (0.40 g). m.p.274-277 °C.

Example 8

6,8-Difluoro-1-(2,4-difluorophenyl)-5-methyl-7-(3-methyl-1-piperazinyl)-1,4-dihydro-4-oxoquinoline-3-carboxylic acid:

6,7,8-Trifluoro-1-(2,4-difluorophenyl)-5-methyl-1,4-dihydro-4-oxoquinoline-3-carboxylic acid (0.50 g) and 2-methylpiperazine (0.50 g) were allowed to react in the same manner as described in Example 1 to give 6,8-difluoro-1-(2,4-difluorophenyl)-5-methyl-7-(3-methyl-1-piperazinyl)-1,4-dihydro-4-oxoquinoline-3-cabboxylic acid (0.32 g). m.p. 236-238 °C.

Example 9

6,8-Difluoro-1-(2,4-difluorophenyl)-5-methyl-7-(cis-3,5-dimethyl-1-piperazinyl)-1,4-dihydro-4-oxoquinoine-3-carboxylic acid:

6,7,8-Trifluoro-1-(2,4-difluorophenyl)-5-methyl-1,4-dihydro-4-oxoquinoline-3-carboxylic acid (0.50 g) and cis-2,6-dimethylpiperazine (0.50 g) were allowed to react inthe same manner as described in Example 1 to give 6,8-difluoro-1-(2,4-difluorohenyl)-5-methyl-7-(cis-3,5-dimethyl-1-piperazinyl)-1,4-dihydro-4-oxoquinoline-3-carboxylic acid (0.51 g). m.p. 134-136 °C, 240-243 °C.

Example 10

1-Cyclopropyl-6-fluoro-5-methyl-7-(1-piperazinyl)-1,4-dihydro-4-oxoquinoline-3-carboxylic acid:

A mixture of 1-cyclopropyl-6,7-difloro-5-methyl-1,4-dihydro-4-oxoquinoline-3-carboxylic acid (300 mg), anhydrous piperazine (660 mg) and pyridine (5 ml) was refluxed for 45 minutes. The solvent was evaporated under reduced pressure. Acetonitrile was added to the residue and cooled with ice. The precipitated crystals were filtered and dissolved in 4 % aqueous sodium hydroxide. The solution was adjusted to pH 8 with 10 % aqueous acetic acid and cooled with ice. The precipitated crystals were collected, washed with water and dried to give 1-cyclopropyl-6-fluoro-5-methyl-7-(1-piperazinyl)-1,4-dihydro-4-oxoquinoline-3-carboxylic acid (332 mg). m.p. 223-234 °C.

In the same manner as described in Examle 10 compounds of the following Examples 11 to 15 were obtained.

Example 11

1-Cyclopropyl-6-fluoro-5-methyl-7-(4-methyl-1-piperazinyl)-1,4-dihydro-4-oxoquinoline-3-carboxylic acid. m.p. 229-230 °C.

Example 12

1-Cyclopropyl-6-fluoro-5-methl-7-(3-methyl-1-piperazinyl)-1,4-dihydro-4-oxoquinoline-3-carboxylic acid. m.p. 208-211 °C.

Example 13

1-Cyclopropyl-6-fluoro-5-methyl-7-(cis-3,5-dimethyl-1-piperazinyl)-1,4-dihydro-4-oxoquinoline-3-carboxylic acid. m.p. 245-246 °C.

Example 14

7-(3-Amino-1-pyrrolidinyl)-1-cyclopropyl-6-fluoro-5-methyl-1,4-dihydro-4-oxoquinoline-3-carboxylic acid. m.p. 268-271 °C.

Example 15

7-(3-Aminomethyl-1-pyrrolidinyl)-1-cyclopropyl-6-fluoro-5-methyl-1,4-dihydro-4-oxoquinoline-3-carboxylic acid. m.p. 207-210 °C.

Example 16

1-Cyclopropyl-6-fluoro-5-methyl-7-(1-piperazinyl)-1,4-dihydro-4-oxoquinoline-3-carboxylic acid:

(1) 1-Cyclopropyl-6,7-difluoro-5-methyl-1,4-dihydro-4-oxoquinoline-3-carboxylic acid and 1-acethylpiperazine were allowed to react in the same manner as described in Example 10 to give 7-(4-acetyl-1-piperazinyl)-1-cyclopropyl-6-fluoro-5-methyl-1,4-dihydro-4-oxoquinoline-3-carboxylic acid. m.p. 276-277 °C.

(2) A mixture of the above compound and 20 % hydrochloric acid was refluxed for 5 hours. The mixture was treated with activated carbon and evaporated under reduced pressure. Acetonitrile was added to the residue and the precipitated crystals were filtered off to give 1-cyclopropyl-6-fluoro-5-methyl-7-(1-piperazinyl)-1,4-dihydro-4-oxoquinoline-3-carboxylic acid. m.p. 233-234 °C.

Referential Example 4

7-(4-Acetyl-1-piperazinyl)-8-amino-1-cyclopropyl-6-fluoro-5-methyl-1,4-dihydro-4-oxoquinoline-3-carbox ylic acid:

(1) To a mixture of 7-(4-acetyl-1-piperazinyl)-1-cyclopropyl-6-fluoro-5-methyl-1,4-dihydro-4-oxoquinoline-3-carboxylic acid (20 g) and acetic ahhydride (200 ml) was added a mixted solution of fuming nitric acid (40 ml) and acetic anhydride (80 ml) dropwise at below 12 °C. under ice-cooling. The mixture was stirred for 30 minutes and ice water was added. The precipitated crystals were collected and washed with water. Recrystallization from chloroform-ethanol gave 7-(4-acetyl-1-piperazinyl)-1-cyclopropyl-6-fluoro-5-methyl-8-nitro-1,4-dihydro-4-oxoquinoline-3-carboxylic acid (13.3 g). m.p. 243-244 °C.

(2) The above compound (0.55 g) in glacial acetic acid (20 ml) was hydrogenated at 60 °C in the presence of 5 % palladium-carbon (80 mg) as catalyst. After a theoretical amount of hydrogen was absorbed, the catalyst was filtered off and the filtrate was evaporated to dryness under reduced pressure. Water and chloroform were added to the residue and the chloroform layer was separated and evaporated under reduced pressure. Recrystallization of the residue from chloroform-ethanol gave 7-(4-acetyl-1-piperazinyl)-8-amino-1-cyclopropyl-6-fluoro-5-methyl-1,4-dihydro-4-oxoquinoline-3-carboxylic acid (0.35 g). m.p. 140-142 °C.

Referential Example 5

8-Amino-1-cyclopropyl-6-fluoro-5-methyl-7-(4-methyl-1-piperazinyl)-1,4-dihdro-4-oxoquinoline-3-carboxylic acid:

(1) 1-Cyclopropyl-6-fluoro-5-methyl-7-(4-methyl-1-piperazinyl)-1,4-dihydro-4-oxoquinoline-3-carboxylic acid (1.7 g) was treated with fuming nitric acid (3.4 ml) and acetic anhydride (21 ml) in the same manner as described in Referential Example 4 (1) to give 1-cyclopropyl-6-fluoro-5-methyl-7-(4-methyl-1-piperazinyl)-8-nitro-1,4-dihydro-4-oxoquinoline-3-carboxylic acid (0.7 g), which was recrystallized from chloroform-ethanol. m.p. 238-240 °C.

(2) The above compound (0.61 g) was hydrogenated in the same manner as described in Referential Example 4(2) to give 8-amino-1-cyclopropyl-6-fluoro-5-methyl-7-(4-methyl-1-piperazinyl)-1,4-dihydro-4-oxoquinoline-3-carboxylic acid (0.38 g). m.p. 211-212 °C.

Example 17

8-Chloro-1-cyclopropyl-6-fluoro-5-methyl-7-(1-piperazinyl)-1,4-dihydro-4-oxoquinoine-3-carboxylic acid:

(1) A mixture of t-butyl nitrite (0.42 g) and acetonitrile (30 ml) was heated at 65 °C and cuprous chloride (0.38 g) was added. To this mixture was added 7-(4-acetyl-1-piperazinyl-8-amino-1-cyclopropyl-6-fluoro-5-methyl-1,4-dihydro-4-oxoquinoline-3-carboxylic acid (1.0 g), and the mixture was stirred at 65 °C for 35 minutes. After the insoluble materials were filtered off, the filtrate was evaporated to dryness under reduced pressure, and water and chloroform were added to the residue. The chloform layer was separated, dried over sodium sulfate and evaporated under reduced pressure. The residue was purified by column chromatography to give 7-(4-acetyl-1-piperazinyl)-8-chloro-1-cyclopropyl-6-fluoro-5-methyl-1,4-dihydro-4-oxoquinoline-3-carboxylic acid (0.31 g), which was recrystallized from chloroform-ethanol. m.p. 214-215 °C.

(2) A mixture of the above compound (0.25 g) and 15 % hydrochloric acid was refluxed for 5 hours. The mixture was treated with activated carbon and evaporated under reduced pressure. The residue was recrystallized from ethanol-water to give 8-chloro-1-cyclopropyl-6-fluoro-5-methyl-7-(1-piperazinyl)-1,4-dihydro-4-oxoquinoline-3-carboxylic acid (0.16 g). m.p.>300 °C.

Example 18

8-Chloro-1-cyclopropyl-6-fluoro-5-methyl-7-(4-methyl-1-piperazinyl)-1,4-dihydro-4-oxoquinoline-3-carboxylic acid:

8-Amino-1-cyclopropyl-6-fluoro-5-methyl-7-(4-methyl-1-piperazinyl)-1,4-dihydro-4-oxoquinoline-3-carboxylic acid (1.8 g) was treated in the same manner as described in Example 17 (1) to give 8-chloro-1-cyclopropyl-6-fluoro-5-methyl-7-(4-methyl-1-piperazinyl)-1,4-dihydro-4-oxoquinoline-3-carboxylic acid 0.62 g). m.p. 225-227 °C (decompn.).

Example 19

1-Cyclopropyl-6,8-difluoro-5-methyl-7-(4-methyl-1-piperazinyl)-1,4-dihydro-4-oxoquinoline-3-carboxylic acid:

To a mixtur of 8-amino-1-cyclopropyl-6-fluoro-5-methyl-7-(4-methyl-1-piperazinyl)-1,4-dihydro-4-oxoquinoline-3-carboxylic acid (0.2 g) and IN hydrochloric acid (33 ml), cooled at 0 °C, was added a solution of sodium nitrite (0.43 g) in water (2 ml). To this mixture was added 60 % hexafluorophosphoric acid (2 ml) at below 5 °C, and the mixture was stirred for 10 minutes. The precipitated crystals were collected and washed successively with ethanol, chloroform and ether. a mixture of the crystals and n-hexane (50 ml) was heated at 80 °C for 30 minutes with stirring. The crystals were collected, and water and chloroform were added. After the water layer was neutralized, the chloroform layer was separated and dried over sodium suflate. The solvent was evaporated under reduced pressure, and the residue was purified by column-chromatography to give 1-cyclopropyl-6,8-difluoro-5-methyl-7-(4-methyl-1- piperazinyl)-1,4-dihydro-4-oxoquinoline-3-carboxylic acid (0.07 g). m.p. 228-229 °C.

Examples 20 to 22 illustrate pharmaceutical compositions containing the compounds of the invention as active ingredients.

Example 20

| | |
|---|---|
| Compound of this invention | 250 g |
| Starch | 50 g |
| Lactose | 35 g |
| Talc | 15 g |

The above components were blended with ethanol and granulated and filled into 1,000 capsules in accordance with conventional methods.

Example 21

| | |
|---|---|
| Compound of this invention | 250 g |
| Starch | 54 g |
| Calcium carboxymethyl cellulose | 40 g |
| Microcrystalline cellulose | 50 g |
| Magnesium stearate | 6 g |

The above components were blended with ethanol, granulated and made into tablets in a manner known per se. Thus, 1,000 tablets each weighing 400 mg were formed.

Example 22

| | |
|---|---|
| Compound of this invention | 50 g |
| Lactic acid | 120 g |

The above components were dissolved in distilled water sufficient to make ten liters solution. The solution was adjusted to pH about 4 with an aqueous sodium hydroxide solution, and then filled in ampules (10 ml) to make an injectable solution.

The chemotherapeutic activities and some other properties of the compounds of this invention are shown in Examples 23-28 hereinbelow. The compounds tested comprise:

Compound 1: 1-cyclopropyl-6,8-difluoro-5-methyl-7-(cis- 3,5-demethyl-1-piperazinyl)-1,4-dihydro-4-oxoquinoline-3-carboxylic acid,

Compound 2: 1-cyclopropyl-6,8--difluoro-5-methyl-7-(1-piperazinyl)-1,4-dihydro-4-oxoquinoline-3-carboxylic acid.

Compound 3: 7-(3-amino-1-pyrrolidinyl)-1-cyclopropyl-6,8-difluoro-5-methyl-1,4-dihydro-4-oxoquinoline-3-carboxylic acid,

Compound 4: 1-cyclopropyl-6-fluoro-5-methyl-7-(cis-3,5-dimethyl-1-piperazinyl)-1,4-dihydro-4-oxoquinoline-3-carboxylic acid,

Compound 5: 1-cyclopropyl-6-fluoro-5-methyl-7-(1-piperazinyl)-1,4-dihydro-4-oxoquinoline-3-carboxylic acid,

Compound A: 1-cyclopropyl-6-fluoro-7-(1-piperazinyl)-1,4-dihydro-4-oxoquinoline-3-carboxylic acid hydrochloride [ciprofloxacin],

Compound B: 1-cyclopropyl-6,8-difluoro-7-(1-piperazinyl)-1,4-dihydro-4-oxoquinoline-3-carboxylic acid,

Compound C: 1-cyclopropyl-6,8-difluoro-7-(cis-3,5-dimethyl-1-piperazinyl)-1,4-dihydro-4-oxoquinoline-3-carboxylic acid,
Compound D: 8-cyano-1-cyclopropyl-6-fluoro-5-methyl-7-(1-piperazinyl)-1,4-dihydro-4-oxoquinoline-3-carboxylic acid.

Example 23

The antibacterial activity in vitro is shown in Table 1. The figures in the table show minimum inhibitory concentrations (MIC) (μg/ml), calculated for free base. The minimum inhibitory concentration was determined by the twofold agar-dilution method, which was recommended by Japan Society of Chemotherapy (Chemotherapy, 29(1), 76(1981)), using Mueller-Hinton agar. One loopful of an overnight culture of test organisms in Mueller-Hinton broth was inoculated onto 10 ml drug- containing agar layers in petri dishes. Bacterial inocula contained approximately $10^6$ colonyl-forming units. Bacterial growth was observed after 20-hour incubation at 37 °C. The MIC was defined as the lowest drug concentration which prevented visible bacterial growth.

Table 1. In vitro antibacterial activity

| Strain | | 1 | 2 | 3 | 4 | 5 | A | B | C | D |
|---|---|---|---|---|---|---|---|---|---|---|
| Gram + | S. aureus 209 JC-1 | 0.05 | 0.025 | 0.0125 | 0.025 | 0.05 | 0.1 | 0.1 | 0.1 | 0.1 |
| | S. aureus Terajima | 0.05 | 0.025 | 0.0125 | 0.025 | 0.025 | 0.1 | 0.1 | 0.1 | 0.1 |
| | S. aureus No. 80 | 0.0125 | 0.0125 | 0.0125 | 0.05 | 0.0125 | 0.39 | 0.2 | 0.1 | 0.1 |
| | S. epidermidis No. 8 | 0.025 | 0.025 | 0.0125 | 0.05 | 0.025 | 0.1 | 0.1 | 0.1 | 0.1 |
| | S. pyogenes A65 | 0.2 | 0.39 | 0.2 | 0.1 | 0.39 | 0.2 | 0.39 | 0.78 | 1.56 |
| Gram - | E. coli NIHJ JC-2 | 0.025 | 0.0063 | 0.0125 | 0.0125 | 0.0125 | 0.0063 | 0.0063 | 0.0125 | 0.0125 |
| | E. coli P-5101 | 0.025 | 0.0063 | 0.0125 | 0.025 | 0.0125 | 0.0063 | 0.0063 | 0.0125 | 0.0125 |
| | S. marcescens S-9 | 0.39 | 0.1 | 0.05 | 0.39 | 0.2 | 0.05 | 0.05 | 0.2 | 0.39 |
| | P. aeruginosa 12 | 0.2 | 0.2 | 0.2 | 0.2 | 0.1 | 0.1 | 0.1 | 0.78 | 0.39 |

Example 24

The antichlamydia activity is shown in Table 2. The figures in the table show minimum inhibitory concentrations (MIC), (µg/ml), calculated for free base.

Minimum inhibitory concentrations (MIC) were determined as follows. McCoy cells were freshly cultured in Eagle's minimum essential medium (EMEM) (Flow) supplemented with 4 % fetal bovine serum and 0.03 % L-glutamine. The cells were trypsinized and suspended in the same culture medium at a cell concentration of 1-2 x $10^5$ cells/ml. One ml of the cell suspension was pipetted into flat-bottomed plastic tubes (14 mm in diameter) containing a cover slip (12 mm in diameter). The tubes were incubated in 5 % $CO_2$-air at 36 °C for 20 hours. One-half ml of a chlamydial suspension (ca. 1-4 x $10^3$ inclusion body forming units) was added into each tube, which was then centrifuged at 1500 x g for 60 minutes. The tubes were incubated for 1 hour at 36 °C. The medium was changed with 1 ml of EMEM supplemented with 8 % fetal bovine serum, 0.03 % L-glutamine, 1 µg/ml cycloheximide and 0.5 % glucose, and containing drugs at various concentrations. After further incubation at 36 °C for 40-48 hours, the cells on the cover glasses were stained with the Giemsa's solution. Inclusion bodies in the cells on the cover glasses were observed with a microscope at a magnification of 200-400. The MIC was defined as the lowest drug concentration where there were no inclusion bodies in all the cells on the cover slip.

Table 2. Antichlamydia activity

| Compound / Organism | 1 | 2 | 3 | A | B | C | D |
|---|---|---|---|---|---|---|---|
| C. Trachomatics G/ur 931 | 0.013 | 0.05 | 0.013 | 1 | 0.39 | 0.2 | 1.56 |
| C. Trachomatics G/ur 1242 | 0.025 | 0.10 | 0.013 | 1 | 0.39 | 0.2 | 3.13 |
| C. Trachomatics G/ur 1317 | 0.013 | 0.10 | 0.013 | 2 | 0.39 | 0.2 | 3.13 |
| C. Trachomatics G/ur 1448 | 0.025 | 0.10 | 0.013 | 1 | 0.39 | 0.2 | 3.13 |

Example 25

The antimycoplasma activity is shown in Table 3. The figures in the table show minimum inhibitory concentrations (MIC), (µg/ml), calculated for free base.

Minimum inhibitory concentrations (MIC) were determined by the twofold agar dilution method. The media used were Chanock broth and agar [PPLD broth and agar (Difco) supplemented with 20 % horse serum and 10 % fresh yeast extract]. A 2-3 day broth culture of organisms was diluted with Chanock broth

to a cell density of about $10^6$ cells/ml. One loopful (about 1 micrometer) of the organism dilution was spotted onto 10-ml drug containing Chanock agar in petri dishes using a multiple inoculator (Cathra International). The petri dishes were incubated at 37 °C for 7 and 2 days for Mycoplasma pneumoniae and other Mycoplasma spp. respectively. Incubation was performed anaerobically using the Gaspak anaerobic system (BBL) for M. buccale, M. fermentans, M. hominis, M. orale and M. salivarium, and aerobically for other Mycoplasma spp. The MIC was defined as the lowest compound concentration at which no growth of organism was detected.

Table 3. Antimycoplasma activity

| Compound / Organism | 1 | A | C |
|---|---|---|---|
| M. pneumoniae Mac | 0.05 | 0.78 | 0.2 |
| A. laidlawii PG-8 | 0.05 | 0.39 | 0.39 |
| M. arginini G-230 | 0.025 | 0.39 | 0.39 |
| M. buccale CH-20249 | 0.025 | 0.39 | 0.2 |
| M. fermentans PG-18 | 0.025 | 0.2 | 0.1 |
| M. hominis PG-21 | 0.05 | 1.56 | 0.2 |
| M. orale CH-19299 | 0.2 | 1.56 | 0.78 |
| M. salivarium PG-20 | 0.1 | 3.13 | 0.78 |
| M. hyorhinis BST-7 | 0.025 | 0.39 | 0.1 |

Example 26

In vivo efficacy against systemic infections in mice is shown in Table 4.

Compounds were each suspended in 0.4 % carboxymethyl cellulose. Each of the suspensions was orally administered to mice infected with each of the test organisms under the conditions shown hereinbelow, and the median effective dose ($ED_{50}$) was calculated by probit analysis. The numerals in the table show $ED_{50}$ (mg/kg) value, calculated for free base.

Experimental conditions:

Mice: Male mice (Std-ddY) weighing about 20 g

Infection:

Streptococcus pyogenes A65

Intraperitoneal infection with 3 x $10^7$ cells per mouse suspended in brain heart infusion broth.

Pseudomonas aeruginosa 12

Intraperitoneal infection with about 5 x $10^3$ cells per mouse suspended in tryptosoy broth with 4 % mucin.

Medication:

Twice, immediately and 6 hours after infection.

Observation:

For 7 days.

Table 4. In vivo efficacy against systemic infections in mice

| Organism \ Compound | 1 | 2 | 4 | A | B | D |
|---|---|---|---|---|---|---|
| S. pyogenes A65 | 5.01 | 8.36 | 7.74 | 23.9 | 13.0 | >50 |
| P. aeruginosa 12 | 1.46 | 1.05 | 1.88 | 2.78 | 1.22 | 1.64 |

Example 27

A suspension containing each of compounds of this invention in various concentrations was orally given to male mice (ddY) at a dose of 0.1 ml per 10 g of body weight. The number of dead mice was counted after 7 days, and the value of median lethal dose ($LD_{50}$, mg/kg) was calculated in accordance with the Behrens-Kaerber method. The results are shown in Table 5.

Table 5

| Acute oral toxicity in mice | |
|---|---|
| Compound | $LD_{50}$ (mg/kg) |
| 1 | >2000 |
| 2 | >2000 |
| 3 | >2000 |
| 4 | >2000 |
| 5 . | >2000 |

Example 28

A suspension containing Compound 1 was orally administered to mace at a dose of 5 mg/kg. Urine was collected over a period of 24 hours after administration. The level of the compound in urine was determined by the thin-layer cup-plated method using Escherichia coli kp as an indicator organism.

Table 6

| Urinary excretion in mice | |
|---|---|
| Compound | 1 |
| Concentration | 7.5 g/ml |
| Volume | 14.0 ml |
| Amount | 0.105 mg |
| Urinary recovery for 24 hours | 12.7 % |

**Claims**

1. A quinoline derivative of the formula

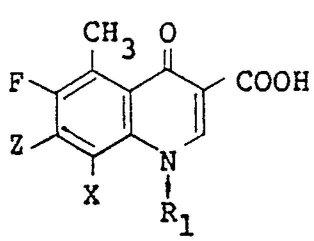

(I)

wherein $R_1$ is a lower alkyl group having 1 to 5 carbon atoms, a cycloalkyl group having 3 to 6 carbon atoms, or an optionally substituted phenyl group, X is a hydrogen atom or a halogen atom, Z is

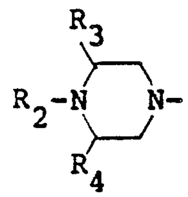

or

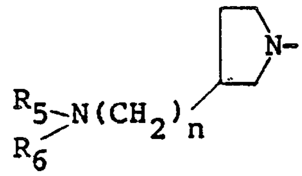

in which $R_2$, $R_3$, $R_4$, $R_5$ and $R_6$ are the same or different and each represents a hydrogen atom or a lower alkyl group having 1 to 5 carbon atoms, and n is 0 or 1,
or a pharmaceutically acceptable ester thereof or a pharmaceutically acceptable salt of said derivative or ester.

2. A compound as claimed in claim 1 wherein $R_1$ is a cyclopropyl group.

3. A compound as claimed in claim 1 wherein X is a fluorine atom.

4. 1-Cyclopropyl-6,8-difluoro-5-methyl-7-(cis-3,5-dimethyl-1-piperazinyl)-1,4-dihydro-4-oxoquinoline-3-carboxylic acid and its pharmaceutically acceptable acid addition salt.

5. 1-Cyclopropyl-6,8-difluoro-5-methyl-7-(1-piperazinyl)-1,4-dihydro-4-oxoquinoline-3-carboxylic acid and its pharmaceutically acceptable acid addition salt.

6. 1-Cyclopropyl-6-fluoro-5-methyl-7-(cis-3,5-demethyl-1-piperazinyl)-1,4-dihydro-4-oxoquinoline-3-carboxylic acid and its pharmaceutically acceptable acid addition salt.

7. 1-Cyclopropyl-6-fluoro-5-methyl-7-(1-piperazinyl)-1,4-dihydro-4-oxoquinoline-3-carboxylic acid and its pharmaceutically acceptable acid addition salt.

8. A pharmaceutical composition comprising a compound defined in claim 1 as an active ingredient and a pharmaceutically acceptable carrier.

9. A method for treatment of a bacterial infectious desease which comprises administering an effective amount of a compound defined in claim 1 to a warm-blooded animal.

10. Use of a compound defined in claim 1 for treatment of a bacterial infectious desease.

11. A process for preparing a quinoline derivative of the formula

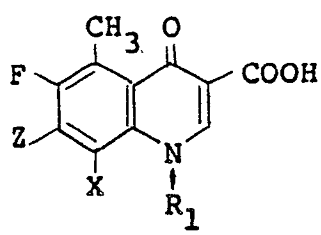

(I)

wherein $R_1$ is a lower alkyl group having 1 to 5 carbon atoms, a cycloalkyl group having 3 to 6 carbon atoms, or an optionally substituted phenyl group, X is a hydrogen atom or halogen atom, Z is

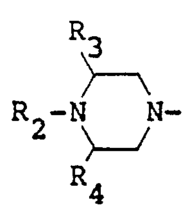

or

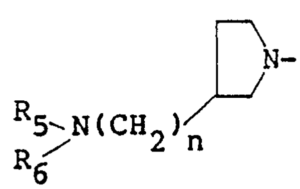

in which $R_2$, $R_3$, $R_4$, $R_5$, and $R_6$ are the same or different and each represents a hydrogen atom or a lower alkyl group having 1 to 5 carbon atoms, and n is 0 or 1,
or a pharmaceutically acceptable ester thereof or a pharmaceutically acceptable salt of said derivative or ester which comprises

(i) reacting a compound of the formula

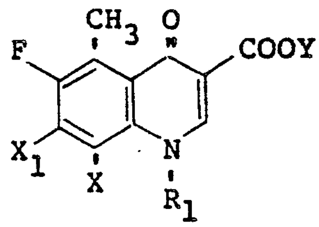

(II)

wherein $X_1$ is a halogen atom, Y is a hydrogen atom or a lower alkyl group having 1 to 5 carbon atoms, and $R_1$ and X are as defined above,
with a compound of the formula

Z-H (III)

wherein Z is as defined above,
and, if desired, hydrolyzing the resulting compound,

(ii) diazotizing a compound of the formula

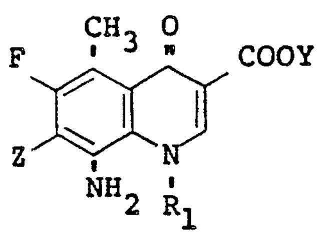

(IV)

wherein $R_1$, Y and Z are as defined above,
and halogenating the so-obtained diazonium compound and, if desired, hydrolyzing the resulting compound, or

(iii) removing the protective group of a compound of the formula

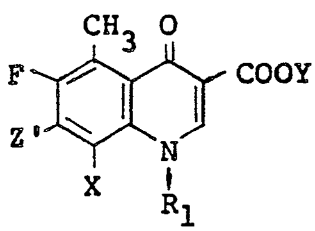

(V)

wherein $R_1$, X and Y are as defined above, and Z' is

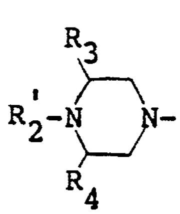

or

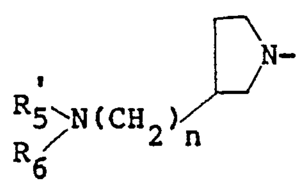

in which $R_2'$ and $R_5'$ represent a protective group, and $R_3$, $R_4$, $R_6$ and n are as defined above.
and, if desired, hydrolyzing the resulting compound,
and

(iv) if desired, converting the thus prepared compound into a pharmaceutically acceptable salt thereof.

12. A process for preparing a quinoline derivative of the formula

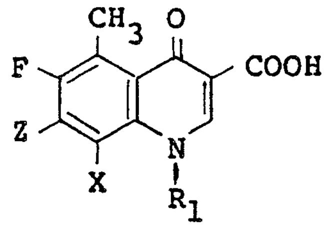

(I)

wherein $R_1$ is a lower alkyl group having 1 to 5 carbon atoms, a cycloalkyl gorup having 3 to 6 carbon atoms, or an optionally substituted phenyl group, X is a hydrogen atom or a halogen atom, Z is

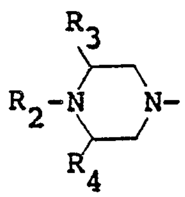

or

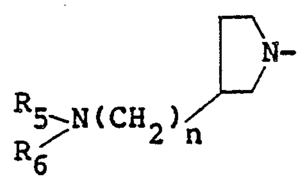

in which $R_2$, $R_3$, $R_4$, $R_5$ and $R_6$ are the same or different and each represents a hydrogen atom or a lower alkyl group having 1 to 5 carbon atoms,
and n is 0 or 1,
or a pharmaceutically acceptable ester thereof or a pharmaceutically acceptable salt of said derivative or ester which comprises reacting a compound of the formula

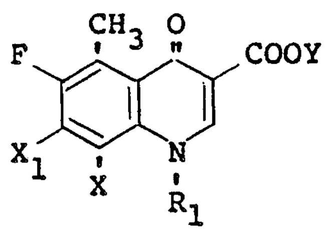

(II)

wherein $X_1$ is a halogen atom, Y is a hydrogen atom or a lower alkyl group having 1 to 5 carbon atoms, and $R_1$ and X are as defined above,
with a compound of the formula

Z-H (III)

wherein Z is as defined above,
and, if desired, hydrolyzing the resulting compound, and optionally converting the thus prepared compound into a pharmaceutically acceptable salt thereof.

13. A process for preparing a quinoline derivative of the formula

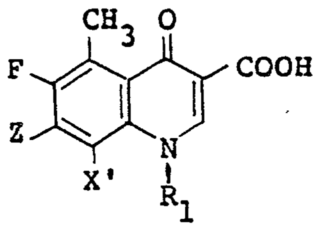

(I')

wherein $R_1$ is a lower alkyl group having 1 to 5 carbon atoms, a cycloalkyl gorup having 3 to 6 carbon atoms, or an optionally substituted phenyl group, X′ is a halogen atom, Z is atom, Z is

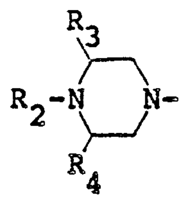

or

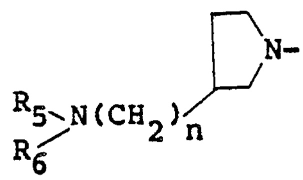

in which $R_2$, $R_3$, $R_4$, $R_5$ and $R_6$ are the same or different and each represents a hydrogen atom or a lower alkyl group having 1 to 5 carbon atoms, and n is 0 or 1, or a pharmaceutically acceptable ester thereof or a pharmaceutically acceptable salt of said derivative or ester which comprises diazotizing a compound of the formula

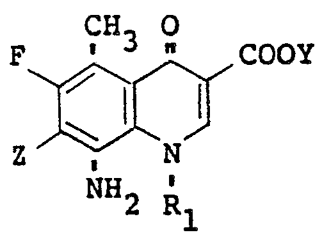

(IV)

wherein $R_1$ and Z are as defined above, and Y is a hydrogen atom or a lower alkyl group having 1 to 5 carbon atoms,
and halogenating the so-obtained diazonium compound and, if desired, hydrolyzing the resulting compound, and optionally converting the thus prepared compound into a pharmaceutically acceptable salt thereof.

14. A process for preparing a quinoline derivative of the formula

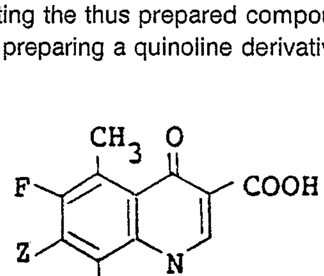

(I)

wherein $R_1$ is a lower alkyl group having 1 to 5 carbon atoms, a cycloalkyl gorup having 3 to 6 carbon atoms, or an optionally substituted phenyl group, X is a hydrogen atom or a halogen atom, Z is

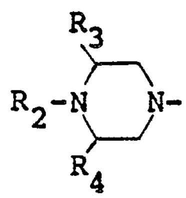

or

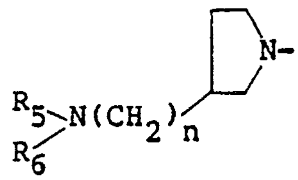

in which $R_2$ and $R_5$ represent a hydrogen atom, $R_3$, $R_4$ and $R_6$ are the same or different and each represents a hydrogen atom or a lower alkyl group having 1 to 5 carbon atoms, and n is 0 or 1,
or a pharmaceutically acceptable ester thereof or a pharmaceutically acceptable salt of said derivative or ester which comprises removing the protective group of a compound of the formula

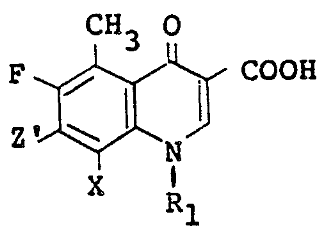

(V)

wherein $R^1$ and X are as defined above, Y is a hydrogen atom or a lower alkyl group having 1 to 5 carbon atoms, and $Z'$ is

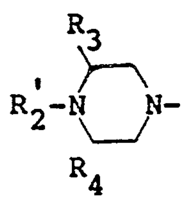

or

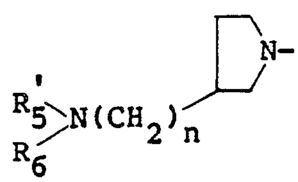

in which $R_2'$ and $R_5'$ represent a protective group, and $R_3$, $R_4$, $R_6$ and n are as defined above.
and, if desired, hydrolyzing the resulting compound, and optionally converting the thus prepared compound into a pharmaceutically acceptable salt thereof.